# EUROPEAN PATENT SPECIFICATION

(11) **EP 2 982 241 B1**
(45) Date of publication and mention of the grant of the patent: **04.12.2019**
(21) Application number: 14179966.8
(22) Date of filing: 06.08.2014
(51) Int. Cl.: A01H 3/00, A01H 3/04, A01N 63/04, A01H 17/00, C12N 1/38

(54) **A method of mycorrhization of plants and use of saccharides in mycorrhization**
Verfahren zur Mykorrhizierung von Pflanzen und Verwendung von Sacchariden bei der Mykorrhizierung
Procédé de mycorhization de plantes et utilisation de saccharides dans la mycorhization

(43) Date of publication of application: 10.02.2016
(73) Proprietor: INOQ GmbH, 29465 Schnega (DE)
(72) Inventor: Lucic, Eva, 29594 Soltendieck OT Varbitz (DE); Mercy, Louis, 29594 Soltendieck OT Varbitz (DE)
(74) Representative: Kröncke, Rolf

(56) References cited:
- CA-A1- 1 154 605
- GUERIN-LAGUETTE ALEXIS ET AL: "Effects of experimental conditions on mycorrhizal relationships between Pinus sylvestris and Lactarius deliciosus and unprecedented fruit-body formation of the Saffron milk cap under controlled soilless conditions", CANADIAN JOURNAL OF MICROBIOLOGY, vol. 46, no. 9, September 2000 (2000-09), pages 790-799, XP009181777, ISSN: 0008-4166
- DUCLOS J L ET AL: "EFFECT OF GLUCOSE AND ACTIVE CHARCOAL ON IN-VITRO SYNTHESIS OF ERICOID MYCORRHIZA WITH VACCINIUM-SPP", NEW PHYTOLOGIST, vol. 94, no. 1, 1983, pages 95-102, XP002733917, ISSN: 0028-646X
- GILTRAP N J: "INFLUENCE OF IRRADIANCE AND CONCENTRATION OF GLUCOSE IN THE SUBSTRATE ON MYCORRHIZAL DEVELOPMENT BY CENOCOCCUM-GEOPHILUM AND PAXILLUS-INVOLUTUS IN AXENIC CULTURE", TRANSACTIONS OF THE BRITISH MYCOLOGICAL SOCIETY, vol. 81, no. 3, 1983, pages 627-629, XP002733918, ISSN: 0007-1536
- HUTCHISON LEONARD J ET AL: "Effects of exogenous glucose on mycorrhizal colonization in vitro by early-stage and late-stage ectomycorrhizal fungi", CANADIAN JOURNAL OF BOTANY / JOURNAL CANADIEN DE BOTANIQUE, NATIONAL RESEARCH COUNCIL, OTTAWA, CA, vol. 73, no. 6, 1 January 1995 (1995-01-01), pages 898-904, XP009181776, ISSN: 0008-4026
- SALLY E. SMITH ET AL: "Roles of Arbuscular Mycorrhizas in Plant Nutrition and Growth: New Paradigms from Cellular to Ecosystem Scales", ANNUAL REVIEW OF PLANT BIOLOGY, vol. 62, no. 1, 2 June 2011 (2011-06-02), pages 227-250, XP055159190, ISSN: 1543-5008, DOI: 10.1146/annurev-arplant-042110-103846
- Fiona Gibson ET AL: "Establishment of ectomycorrhizas in aseptic culture: effects of glucose, nitrogen and phosphorus in relation to successions", MYCOLOGICAL RESEARCH, vol. 94, no. 2, 1 March 1990 (1990-03-01), pages 166-172, XP055328131, GB ISSN: 0953-7562, DOI: 10.1016/S0953-7562(09)80608-4

## Description

The present invention relates in a first aspect to the use of monosaccharides and/or disaccharides for stimulation of mycorrhization in plants as well as for the production of mycorrhiza-inoculum in plants as defined in the claims. In addition, the present invention relates to the use of monosaccharides and/or disaccharides, optionally, in combination with mycorrhizal inoculum for improving plant performances (growth, yield and survival). In addition, methods for the production of mycorrhizal inoculum in plants as well as for the treatment of plants, in particular crops, as defined in the claims, is provided. Finally, the present invention relates to a composition for stimulation mycorrhization of plants, for the production of mycorrhizal inoculum in plants, for yield of the plants containing monosaccharides and/or disaccharides as well as mycorrhizal inoculum.

### Prior art

Conventional agricultural practices have ensured yield and protection on crop cultivation by an abundant use of inorganic and organic fertilisers and pesticides, but reach its limits by the awareness of its impact on the environment and human health. Today, legislation associated with growing concern to cope phytosanitary problems exist (European Directive 2009/128/EC). Hence, more sustainable management of agricultural ecosystems involving agroecological strategies are increasingly explored. Soil is the first habitat of the plant as it interacts at all stages of its life cycle, Jeffries P, et al.,2003, Biology and Fertility of Soils 37:1-16, and is one of the main reservoir of ecosystem services. Plants live in a myriad of biotic and abiotic interactions with the soil and environment that determine their growth, productivity and life cycle, whereas rhizosphere plays an interface role. There are also opportunities to better explore beneficial soil microorganisms contributing to these ecosystem services for a sustainable crop production, in order to maintain or improve yield, plant resistance while preserving the nutritional and organoleptic qualities.

About 90 % of all plants form a symbiosis with mycorrhizal fungi under natural condition Smith SE and Read DJ. 2008, Mycorrhizal symbiosis. Cambridge, UK: Academic Press. In a mycorrhizal association, the fungus colonises their host plants roots, either intracellular as an arbuscular mycorrhizal (AM) fungi or extracellulary as an ectomycorrhizal fungi. Arbuscular mycorrhizal (AM) fungi have established symbiotic associations with plant roots for over 450 million years, since the appearance of the earliest land plants, suggesting that AM fungi assisted plants in their colonization of land (Redecker D, et al., 2000, Molecular Phylogenetics and Evolution 14, 276-284). AM fungi are essential soil biota which established the predominant mutualistic symbiotic relationship within the roots of more than 200.000 plants species (Smith SE and Read DJ. 2008, see above). The hyphae of the fungus increase the function of the root system, alter the hormone status of the host plant as well as supply the host plant with water and mineral nutrients (Smith SE and Read DJ. 2008, see above). That is, under this mutualistic association, the plant provides the fungus with relatively constant and direct access to carbohydrates, such as hexoses (glucose and fructose) (Bago B, et al., 2003, Plant Physiology 131: 1-11.) In return, the plant gains benefit from the AM fungi by a higher absorptive capacity for water and mineral nutrition, by a combined physical and chemical mechanisms. Mycorrhizal mycelia are able to explore a larger volume of soil than the root system, improving the surface area for nutritive absorption (Hattingh MJ., et al., 1973, Soil Sciences 116: 383-387). Mycorrhiza are especially beneficial for the plant partner under poor mineral soil values, but also when plants are subject to other abiotic stress (chilling, heat, salty soils, drought, wounding) (Singh LP., et. A., 2011, Plant Signaling & Behavior 6: 175-191).

Moreover, mycorrhizal plants are more resistant to diseases such as those caused by microbial soil-borne pathogens (Jung SC., et al., 2012, Journal of Chemical Ecology 38: 651-664). In addition, due to the improved capability of mineral uptake, the mycorrhizal fungi can increase the growth and yield of host plants.

That is, beneficial effects of AM fungi to the plant host are multiple, as they i) improve plant growth by a better transfer of inorganic nutritions and water (Smith SE and Read DJ. 2008, see above); ii) increase plant pathogen resistance and plant health (Whipps JM. 2004. Canadian Journal of Botany 82:1198-1227); iii) boost plant photosynthesis; iv) stabilise soil by the excretion of a fungal glycoprotein, glomalin (Rillig MC, Steinberg PD. 2002, Soil Biology and Biochemistry 34:1371-1374; v) alleviate impact of abiotic stresses including salinity and drought (Aroca R, et.al., 2007, New Phytologist 173:808-816; Porras-Soriano A, et.al., 2009. Journal of Plant Physiology 166:1350-1359). In term, AM fungi benefits from plants by a habitat in which they can complete their life cycle, associated with an uptake of photosynthetates (Bago et. Al., 2003, see above).

Many efforts have been conducted to exploit the potential of mycorrhiza, starting with mastering the mycorrhizal inoculum production at various scales. Although several methods are available for the production of mycorrhizal inoculum, the average costs of such inoculum on the market remains prohibitive and limit their use at large scale (Vosatka M, et.al., 2008 In: Varma A, ed. Mycorrhiza, Berlin, Springer Berlin Heidelberg, 419-438; Ijdo M, et.al., 2011, Mycorrhiza 21:1-16; Malusá et.al., 2012, The Scientific World Journal 2012:491206 (http://dx.doi.org/10.1100/2012/491206; Smith FA, Smith SE. 2010. Plant Soil 348:63-79; Smith SE, Smith FA. 2011. Annu Rev Plant Biol. 62: 227-250). The method includes *in vivo* pot production on various substrates, *in vivo* hydro/aeroponic systems and *in vitro* production in solid or liquid medium. Therefore, an application of mycorrhizal inoculum is often limited to horticultural or other controlled crop systems under a greenhouse while field applications are limited. In addition, environmental constrains limit mycorrhizal development within root system since mycorrhizal fungi are not able to develop optimally on soils which contain high phosphorus and nitrogen residues (Smith and Read, 2008, see above). Finally, the quantity and quality of mycorrhizal inoculum, also impacted by the weight and volume, the application methodology, and formulation are determinant factors to the successful development of these fungi in the root systems.

US 4,749,402 describes a method and composition for the enhancement of mycorrhizal development by foliar fertilisation of plants. In JP 09-241112 a method for improving infection rates of mycorrhizal fungi is described wherein algenic acid oligosaccharides are applied singly or together with mycorrhizal fungi. JP 09-224647 discloses vesicular arbuscular mycorrhizal fungi cultured by using a medium prepared by adding algenic acid oligosaccharides obtained by precipitation with ethanol, improving hyphal growth.

That is, an extensive use of mycorrhiza in agriculture is limited, due to the unpredictability of the effect of mycorrhiza on the crops. As identified, the efficiency of the symbiosis between plant and AM fungi mainly occurs under limited nutrient availability soil or substrates, which is typically not the case under field crop in modern agriculture. That is, due to the use of high amounts of mineral fertilisers, AM fungi are less able to develop in root, and often associated to low benefits on plant performance (Smith and Smith, 2011, see above).

Hence, there is an ongoing need for improved methods for producing mycorrhizal fungi and its use in agriculture.

The present invention aims in providing methods in order to improve production of mycorrhizal inoculum in plants as well as methods for improving mycorrhizal development in plants, associated with the enhancement of germination rate of plants seeds, and plant yield.

### Description of the present invention

On a first aspect, the present invention relates to the use of monosaccharides and/or disaccharides for stimulation of mycorrhization in plants according to claim 1. The monosaccharides and/or disaccharides are used in a concentration of 100 ppm or less, preferably between 0,1 and 10 ppm or less. The present inventors recognized that the use of low doses of mono- and/or disaccharides promotes the mycorrhizal development in plants. In addition, it is recognized by the present inventors that the mono- and/or disaccharides increase the number of mycorrhizal propagules during mycorrhizal inoculum production when used at low doses. Hence, the present invention relates in another aspect to the use of monosaccharides and/or disaccharides for the production of mycorrhizal inoculum in plants, wherein the monosaccharides and/or disaccharides are used in a concentration of 100 ppm or less. In particular, using the monosaccharides and/or disaccharides according to the present invention for the production of mycorrhizal inoculum in plants represents a possibility to produce mycorrhizal inoculum in larger quantities at low costs. In contrast to prior art which requires the use of expensive compounds in high quantities, thus, not allowing the production of mycorrhizal inoculum at competitive prices for use in commercial agriculture of crops.

In a further aspect, the present invention relates to the use of monosaccharides and/or disaccharides in combination with or in the presence of mycorrhizal inoculum for improving the yield of the plants, whereby the monosaccharides and/or disaccharides are used at low doses, namely, in concentrations of 100 ppm or less.

As identified, low doses of monosaccharides and/or disaccharides are sufficient to stimulate mycorrhization in plants, to promote mycorrhizal inoculum production as well as increasing the germination rate of plants or the yield of the plants when used together with mycorrhizal inoculum. The concentration is 100 ppm or less, for example 1 ppm or less, e.g 0.01 ppm less.

As used herein, the term "monosaccharides and/or disaccharides" include single type monosaccharides including pentoses and hexoses as well as heptoses. For example, C6 monosaccharides include glucose, mannose, altrose, galactose and fructose as well as psicose, sorbose, tagatose, talose, gulose, and idose as well as C5 monosaccharide including Xylose, Xylulose, arabinose, lyxose, ribulose and ribose.

The disaccharides useful according to the present invention include disaccharides like sucrose, lactolose, lactose, maltose, trehalose and cellobiose.

It is preferred, that monosaccharides are used. The monosaccharides and/or disaccharides may be used as single monosaccharides and/or disaccharides or at least two different types of monosaccharides and/or disaccharides accordingly.

In a preferred embodiment, the monosaccharides and/or disaccharides are selected from glucose and/or fructose.

As used herein, the term "comprise" or "comprising" as well as "contain" or "containing" includes the embodiment of "consist" and "consisting of".

As used herein, the term "plant" includes the embodiments of the whole plant as well as parts of plants including roots, leaves and shoots.

As used herein, the term "yield", express both a quantity and a quality of a given plant organ (leaf, fruit, root or tuber) e.g. which has an economic interest, and correspond also to the quantity of harvested product per given land cultivation. In addition, the term "plant performance" include to improve growth, yield and survival.

The present inventors identifies that the low concentrations as defined in the claims (also identified as low doses representing 0.1 to 100 ppm) of the mono- and/or disaccharides have beneficial effects on mycorrhization as well as of the production of mycorrhizal inoculum.

In addition, it has been recognized that using the monosaccharides and/or disaccharides in combination with mycorrhizal inoculum on plants have beneficial effects on the growth of the plants, i.e. the overall yield of the plants.

The plants according to the invention are cultivated under controlled crop conditions. The plants mycorrhization is stimulated under field crop and hydroponic.

In particular, the plants are selected from Rice (*Oryza sp*), Wheat (*Triticum sp*), Tomato (*Solanum lycopersicum*)*,* Sugar cane (*Saccharum sp*), Maize (Zea *mays*), Potato (*Solanum tuberosum*), Grapevine (*Vitis sp*), Cotton (*Gossypium* sp), Apple tree (*Malus sp*), Banana tree (*Musa sp*), Cassava (*Manihot sp*), Mango tree (*Mangifera indica*)*,* Onion (*Allium cepa*)*,* Coffee tree (*Coffea sp*), Bean (*Phaseolus sp*)*,* Soybean (*Glycine max*)*,* Olive (*Olea europaea*)*,* Tea (*Camellia sinensis*), Orange (*Citrus sinensis*), Yam (*Dioscorea sp*), Lettuce (*Lactuca sativa*), Cacao (*Theobroma cacao*), Ginger (*Zingiber officinale*).

In an embodiment, the monosaccharides and/or disaccharides are applied on/or at the vicinity of the plant roots. In another embodiment of the present invention, the monosaccharides and/or disaccharides are applied simultaneously, separately or sequentially together with mycorrhizal inoculum. For example, the mycorrhizal inoculum is applied on or at the vicinity of the plant roots to be treated while at same time or at a later time point, the monosaccharides and/or disaccharides are applied on or at the vicinity of plant roots or on a different plant organ (i.e. leaves).

For example, the monosaccharides and/or disaccharides can be used in a way that they are applied to the mycorrhizal plants directly to the roots, at the root vicinity or foliarly.

For example, the mycorrhizal inoculum is applied at planting time or during the 4 weeks following the planting of seeds, plants or shoots.

The mycorrhizal inoculum may be selected from spores and/or mycorrhizal roots and/or mycelium. Further, the mycorrhizal inoculum can be used in liquid or solid form. Further, in a preferred embodiment, the monosaccharides and/or disaccharides are applied as liquids or solids. In an alternative embodiment, the monosaccharides and/or disaccharides are applied on the leaves and/or directly to the root or at the vicinity of the root while the mycorrhizal inoculum is applied to the roots, i.e. are applied to the soil.

The methodology is compatible with suitable fertilizer application together with the monosaccharides and/or disaccharides. A skilled person is well aware of suitable fertilisers which may be applied with the monosaccharides and/or disaccharides.

When applying monosaccharides and/or disaccharides in combination with mycorrhizal inoculum, the stress tolerance of the plant, in particular, of the crops, can be increased.

As discussed above, the prior art described that mycorrhization occurs more rapidly by improved fungal colonisation in plants roots growing under nutrition deficient environment. That is, at high levels of phosphates, mycorrhizal development is inhibited and the mycorrhizal benefits decrease becoming in some situation parasitic. Furthermore, it is described that mycorrhizal development is stronger in plants suffering from stress. Plant biotic and abiotic stress is usually a situation which is tried to be avoided in agriculture. However, it is described in literature that mycorrhizal fungi, when present, can colonize more quickly in plants suffering from stress.

The present inventors recognized that using the monosaccharides and/or disaccharides according to the present invention can overcome the problem of high phosphate concentration on mycorrhization. That is, in contrast to the situation described in the art, the use of the monosaccharides and/or disaccharides in low concentrations, in concentration of 100 ppm or less, is beneficial on mycorrhization of plants even at high phosphate concentration. Thus, it is possible, to overcome the present problem of effective mycorrhization at high phosphates content. In addition, by increasing the colonisation and mycorrhization of the plants, a reduction of pesticides is possible. Furthermore, the use of the monosaccharides and/or disaccharides according to the present invention allows to foster or to support the mycorrhization even at low phosphate concentration, i.e. at shortage of phosphates, e.g., shortage of fertilisers. In particular, in field agriculture, the use of the monosaccharides and/or disaccharides according to the present invention, optionally, in combination with the mycorrhizal inoculum allows to stimulate mycorrhization of plants, in particular, crops even at high concentration of phosphates. Thus, the metabolic activity of the symbiotic fungus is increased supporting growth of the plants and, consequently, increasing the yield of the plants.

The establishment of mycorrhizal fungi in root, in relation with plant physiological status, seems to be relied on the implementation of Induced Systemic Response (ISR) (Hause, B., et.al., 2007, Phytochemistry 68: 101-110), while establishment of Systemic Acquired Resistance (SAR) limits mycorrhizal development (De Roman M., et.al., 2010, Journal of Ecology 99: 36-45). These two plant defence responses imply opposite growth regulators interplay, which modulate mycorrhizal development. For example, abscisic acid (ABA) has been described as playing a crucial role for arbuscule formation with positive consequences on mycorrhizal development, Herrera-Medina MJ, et.al., JM. 2007, New Phytologist 175:554-564, and is known to be involved in ISR; Van der Ent S., et.al., 2009 New Phytologist 183: 419-431. ABA is antagonist to the SAR, Yasuda M., et.al., 2008, Plant Cell 20: 1678-1692, whose implementation is mainly mediated by salicylic acid (SA), a growth regulator known to inhibits mycorrhizal development Ozgönen, H., et.al., 2001, Turkish Journal of Agriculture & Forestry, 25: 25-29; Blilou I., et.al., 1999, Journal of Experimental Botany 50: 1663-1668; Herrera-Medina MJ, et.al., 2003, Plant science 164: 993-998. Thus, the idea belong to the use of mono/oligosaccharides is to help mycorrhizal fungi to create a favourable plant physiological context to increase the mycorrhizal susceptibility, by inducing a priming improving ISR in plant without the use of growth regulator. It was shown that glucose increases the ABA content. Mono/oligosaccharides application at low dose act therefore as elicitors, i.e. signals that induce specific plant defence response (priming), promoting mycorrhizal development, optimizing exchanges between plant and AMF and benefiting plant performance.

In an embodiment of the present invention, it is possible to apply the mycorrhizal inoculum only once to the plants while the monosaccharides and/or disaccharides are applied once, twice or more to the plants. In an embodiment of the invention, it is preferred to apply only one time.

The present invention relates further to a method for the production of mycorrhizal inoculum in plants wherein monosaccharides and/or disaccharides are present in a concentration of 100 ppm or less when producing the mycorrhizal inoculum. That is, the present inventors recognized that in the presence of the monosaccharides and/or disaccharides as identified herein, the colonisation and the production of mycorrhizal inoculum in plants is increased and the time may be shortened, thus, it is possible to produce mycorrhizal inoculum cost effective in high amounts.

In another aspect, the present invention relates to a composition for stimulating mycorrhization of plants for the yield of plants. Said composition contains monosaccharides and/or disaccharides for application on plants including plant components (like plant extracts). In addition, the composition contains mycorrhizal inoculum for mycorrhization of plants. For example, the kit or composition contains the monosaccharides and/or disaccharides as a liquid or as a solid to be used in a concentration of 100 ppm or less, i.e. applying 100 mg or less/liter of the monosaccharides and/or disaccharides. Said mycorrhizal inoculum may be provided in solid or liquid formulation to be applied preferably to on the roots or at the vicinity of the roots of the plants.

Said kit is to be used in the method according to present invention allowing to increase the yield of the plants to be treated.

### Brief description of the drawings

Figure 1: Enhancement of mycorrhizal development on potato in vitro plantlets inoculated with *Rhizophagus irregularis* by application in soil (A) or on leaves (B) of D-glucose and D-Fructose. Means with the same letter are not significantly different, F% and M% values were analysed independently.
Figure 2: Enhancement of mycorrhizal development on potato in vitro plantlets inoculated with *Rhizophagus irregularis* by application on leaves (once) of D-glucose through 5 phosphorus concentrations. Means with the same letter are not significantly different; F% and M% values as well as data by phosphorus concentration were analysed independently.
Figure 3: Enhancement of mycorrhizal development on potato *in vitro* plantlets inoculated with various AMF species and treated or not with a D-glucose soil application. Means with the same letter are not significantly different. Data by mycorrhizal strain were analysed independently.
Figure 4: Effect on yield of potato plants inoculated (M) or not (NM) with *Rhizophagus irregularis,* treated or not with D-Glucose (5 ml of a solution containing 100 ppm D-Glucose) on leaves containing 50 ppm phosphorus. Means with the same letter are not significantly different.
Figure 5: Effect on yield of potato plants inoculated (M) or not (NM) with *Rhizophagus irregularis,* treated or not with D-Glucose soil application (100 ppm) on 3 different phosphorus concentrations. Means with the same letter are not significantly different.

The present invention will be described by a way of examples further without limiting the invention there to.

### Examples showing enhancement of mycorrhizal development with the monosaccharide application in soil or plant leaves:

### Example 1: Impact of D-Glucose and D-fructose on mycorrhizal development, when applied in soil or on plant leaves.

### Material and Methods

2 weeks old potato *in vitro* plantlets (cv K19-99-0012 growing on modified MS medium) were transplanted in greenhouse in 500ml pot filled with sterile sand substrate (sterilized twice at 120°C for 6h), containing or not *Rhizophagus irregularis isolate B22-5* (300 spores per plant, inoculated at the vicinity of plant root, with a micro pipette). Monosaccharide treatments consist by spraying on leaves (diluted with sterile osmosed water, one time, on both faces of leaves, with 5 ml of solution just after potato plantation), or by mixing (from powder form) directly in the sterile substrate. Concentrations used are as follow:
- D-Glucose: 0.1 g/L (100 ppm in soil; 0.5 ppm final when applied on leaves)
- D-fructose: 0.01 g/L (10 ppm in soil; 0.1 ppm final when applied on leaves)

Control plant group for foliar treatment consist to spray sterile osmosed water (5 ml, pH 4.91) once on leaves (both faces) without monosaccharide at the time of plantation.

For each treatment, 4 repetitions are performed, plant were fertilized once a week with Hoagland's solution (50 ml per pot) containing low phosphate concentration (0.5mg/L) in order to express mycorrhizal development. Plants were then watered when needed.

Plant roots were harvested after 8 weeks culture and stained by China ink, based on protocol of Vierheilig, H. and Piché, Y. (1998), Z. Pflanzenernaehr. Bodenk., 161: 601-602. doi: 10.1002/jpln.1998.3581610515. Then, the mycorrhizal rate was performed according to the protocol of Trouvelot, A.; et.al., (1986): In : Physiological and Genetical Aspects of Mycorrhizae, V. Gianinazzi-Pearson and S. Gianinazzi (eds.). INRA Ress, Paris, S. 217 - 221, using the Mycocalc software (www2.dijon.inra.fr/mychintec/Mycocalc-prg)/download.html).

Data were analysed with the Statistical Analysis System (v 9.1.3, SAS Institute Inc., Cary, NC, USA) by ANOVA one way. Means were separated using Waller-Duncan Baysian K-ratio test at p<0.05. Percentage values were subjected to arcsin transformation before processing.

### Results

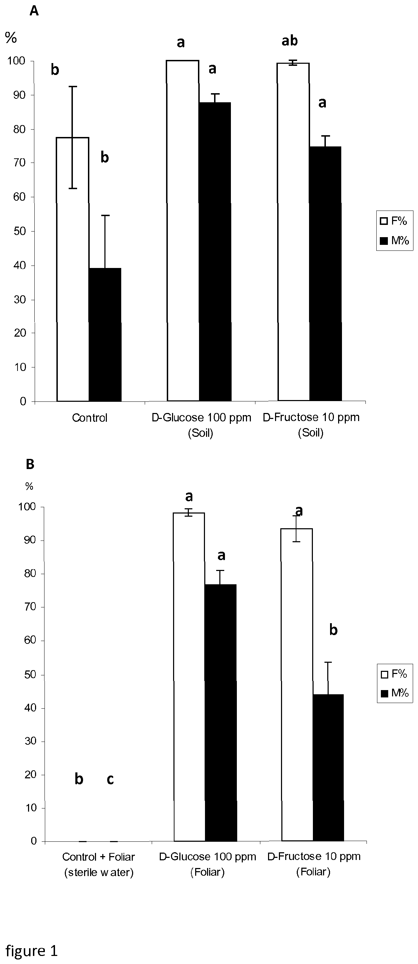

Data on mycorrhizal rate are shown figure 1. The F% (Frequency of mycorrhiza) and M% (intensity of mycorrhizal development in roots) values for the mycorrhizal plants without treatment is respectively 77.5% and 39.07%. No mycorrhizal development was observed from inoculated plants treated with sterile osmosed water. Application of acid water on plant leaves strongly affect photosynthetic capacities, while AMF are obligate biotroph which need photosynthetates (Smith and Read, 2008, see above): the fungus is therefore not able to grow properly within the plant roots.

When monosaccharides are applied in soil, mycorrhizal development is significantly enhanced:
- F% reaches the maximum value 100% (1.29 fold compared to inoculated plants without treatment) and the M% reaches 87.56% (2.24 fold compared to inoculated plants without treatment) after D-glucose application,
- F% reaches 99.17% (1.28 fold compared to inoculated plants without treatment) and the M% reaches 74.26% (1.9 fold compared to inoculated plants without treatment) after D-fructose application.
- Mycorrhizal development is restored when D-glucose or D-fructose is applied on leaves, compared to the inoculated plants treated with sterile osmosed water on leaves. In the case of D-glucose, M% values obtained when plants are treated foliarly are significantly higher than those obtained with inoculated plants without foliar sterile water application.

### Example 2: impact of foliar treatment of D-glucose on mycorrhizal development under various P concentration

### Material and Methods

2 weeks old potato *in vitro* plantlets (cv K19-99-0012 growing on modified MS medium) were transplanted in greenhouse in 500ml pot filled with sterile sand substrate (sterilized twice at 120°C for 6h) containing respectively 10, 50, 100 and 300 ppm of phosphorus concentration (calculation of the different concentrations in ppm is based on the phosphorus present in KH₂PO₄). Plants were inoculated or not with 300 spores of *Rhizophagus irregularis* (INOQ strain QS 69) at the vicinity of roots with micro pipette. Monosaccharide treatments consist by spraying D-glucose on leaves (100 mg/L diluted with sterile tap water at pH7, one time, on both faces of leaves, with 5 ml of solution just after potato plantation, so 0.5 ppm final spray on leaves).

For each treatment, 5 repetitions are performed; plants were fertilized once a week with Hoagland's solution (50 ml per pot) containing no phosphate. Plants were then watered when needed.

Plant roots were harvested after 8 weeks culture and stained by China ink, based on the Vierheilig *et al* (1998) protocol, see above. Then, the mycorrhizal rate was performed according to Trouvelot *et al,* 1986 protocol, see above, using the Mycocalc software (www2.dijon.inra.fr/mychintec/Mycocalc-prj/download.html).

Data were analysed with the Statistical Analysis System (v 9.1.3, SAS Institute Inc., Cary, NC, USA) by ANOVA one way. Means were separated using Waller-Duncan Baysian K-ratio test at p<0.05. Percentage values were subjected to arcsin transformation before processing.

### Results

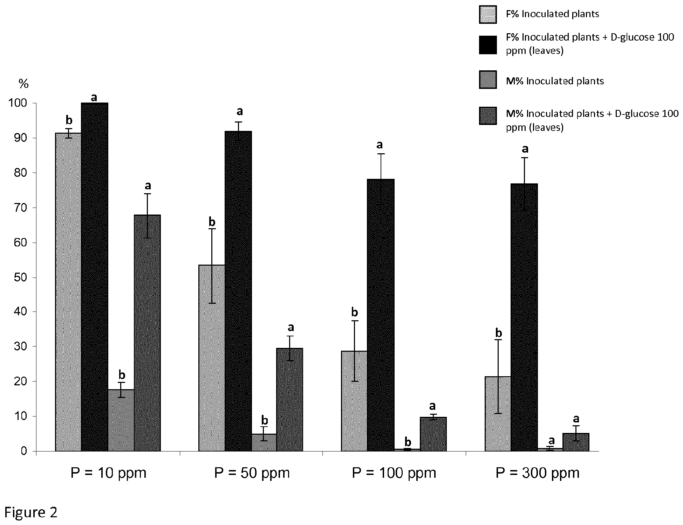

Data on mycorrhizal rate are shown figure 2. The mycorrhizal development is significantly increased by the application on leaves of a D-glucose solution, in all phosphorus concentration tested:
- at 10 ppm phosphorus, D-glucose allow a increase by 1.09 and 3.85 fold, respectively, for F% and M% values.
- at 50 ppm phosphorus, D-glucose allow a increase by 1.72 and 5.96 fold respectively for F% and M% values.
- at 100 ppm phosphorus, D-glucose allow a increase by 2.72 and 16.15 fold respectively for F% and M% values.
- at 300 ppm phosphorus, D-glucose allow a increase by 3.59 and 7.36 fold respectively for F% and M% values.

### Example 3: impact of soil application of D-glucose on different mycorrhizal AMF species development in potato plants

### Material and Methods

2 weeks old potato *in vitro* plantlets (cv K19-99-0012 growing on modified MS medium) were transplanted in greenhouse in 500ml pot filled with sterile sand substrate (sterilized twice at 120°C for 6h). Plants were inoculated with 10 spores of 6 different AMF species (separately), at the vicinity of roots with micro pipette. 4 INOQ *Rhizophagus irregularis* strains were tested and compared with 2 BEG strains as references. AMF species tested were as follow:
- *4 Rhizophagus irregularis strains, noticed a, b, c and d (in vitro spores)*
- *Rhizophagus intraradices BEG 144 (ex vitro spores)*
- *Funneliformis mosseae BEG 12 (ex vitro spores)*

Monosaccharide treatments consist by mixing D-glucose (powder form) directly in the sterile substrate at the concentration of 100 ppm.

For each treatment, 3 repetitions are performed; plants were fertilized once a week with Hoagland's solution (50 ml per pot) containing low phosphate concentration (0.5mg/L). Plants were then watered when needed.

Plant roots were harvested after 8 weeks culture and stained by China ink, based on the Vierheilig *et al* (1998) protocol, see above. Then, the mycorrhizal rate was performed according to Trouvelot *et al,* 1986 protocol, see above, using the Mycocalc software (www2.diion.inra.fr/mychintec/Mycocalc-prg/download.html).

Data were analysed with the Statistical Analysis System (v 9.1.3, SAS Institute Inc., Cary, NC, USA) by ANOVA one way. Means were separated using Waller-Duncan Baysian K-ratio test at p<0.05. Percentage values were subjected to arcsin transformation before processing.

### Results

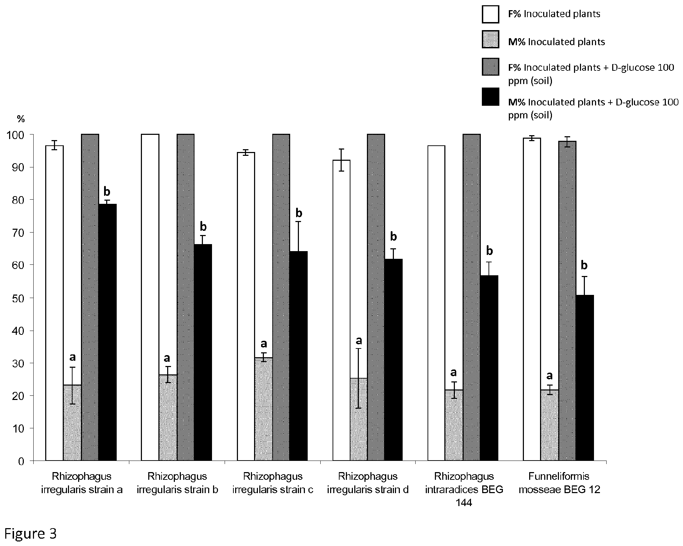

Data on mycorrhizal rate are shown figure 3. Application of D-Glucose in soil has allowed to increase mycorrhizal development, with for the M% (compared to non-treated plants):
- *3.41 fold for Rhizophagus irregularis strain a*
- *2.51 fold for Rhizophagus irregularis strain b*
- *2.02 fold for Rhizophagus irregularis strain cU1*
- *2.45 fold for Rhizophagus irregularis strain dU33*
- *2.61 fold for Rhizophagus intraradices BEG 144*
- *2.33 fold for Funneliformis mosseae BEG 12*

### Examples showing enhancement of tuber yield when plants are inoculated and treated with the monosaccharide:

### Example 1: Foliar application

### Material and Methods

2 weeks old potato *in vitro* plantlets (cv K19-99-0012 growing on modified MS medium) were transplanted in greenhouse in 500ml pot filled with sterile sand substrate (sterilized twice for 6h). Plants were inoculated or not with 300 spores of *Rhizophagus irregularis* (INOQ strain QS 69) at the vicinity of roots with micro pipette. A foliar application with D-glucose (100 ppm diluted with sterile tap water at pH7, one time, on both faces of leaves, with 5 ml of solution just after potato plantation). In this case, in order to obtain tuber yield, plants were fertilized with 50 ppm of phosphorus concentration (ppm is based on the phosphorus added in KH₂PO₄ form), directly mix in the sterile substrate before planting.

For each treatment, 4 repetitions are performed; plants were fertilized once a week with Hoagland's solution (50 ml per pot) containing no phosphate. Plants were then watered when needed.

After 8 weeks culture, tuber were harvested and weighted. Data were analysed with the Statistical Analysis System (v 9.1.3, SAS Institute Inc.,) by ANOVA one way. Means were separated using Waller-Duncan Baysian K-ratio test at p<0.05.

### Results

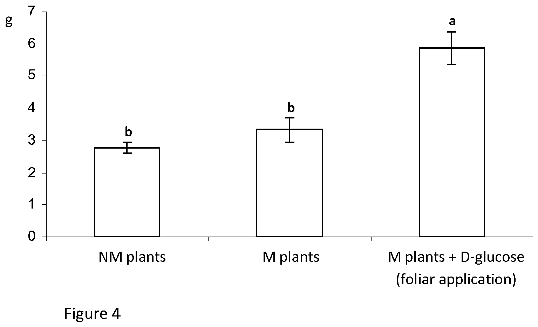

Data on tuber yield obtained after foliar application of D-glucose on potato plants in a soil containing 50 ppm phosphorus is showed Figure 4. Presence of mycorrhizal fungi tends to increase tuber yield by 1.2 fold compared to non-inoculated plants (not significant). Presence of mycorrhizal fungi with a D-glucose application on leaves allows to increase significantly the tuber yield by 2.12 fold compared to non-inoculated plants.

### Example 2: soil application

### Material and Methods

2 weeks old potato *in vitro* plantlets (cv K19-99-0012 growing on modified MS medium) were transplanted in greenhouse in 500ml pot filled with sterile sand substrate (sterilized twice for 6h). Plants were inoculated or not with a mycorrhizal inoculum containing *Rhizophagus irregularis* (INOQ strain QS 69, mix directly in the substrate at 4%). Monosaccharide treatments were as follow:
- A soil application with D-glucose (100 ppm in powder form, mixed directly in the substrate, in the same time than inoculation and just before planting). In this case, in order to obtain tuber yield, plants were fertilized with 50, 100 and 300 ppm of phosphorus concentration (ppm is based on the phosphorus added in KH₂PO₄ form), directly mix in the sterile substrate before planting.

For each treatment, 3 repetitions are performed; plants were fertilized once a week with Hoagland's solution (50 ml per pot) containing no phosphate. Plants were then watered when needed.

After 8 weeks culture, tuber were harvested and weighted. Data were analysed with the Statistical Analysis System (v 9.1.3, SAS Institute Inc.,) by ANOVA one way. Means were separated using Waller-Duncan Baysian K-ratio test at p<0.05.

### Results

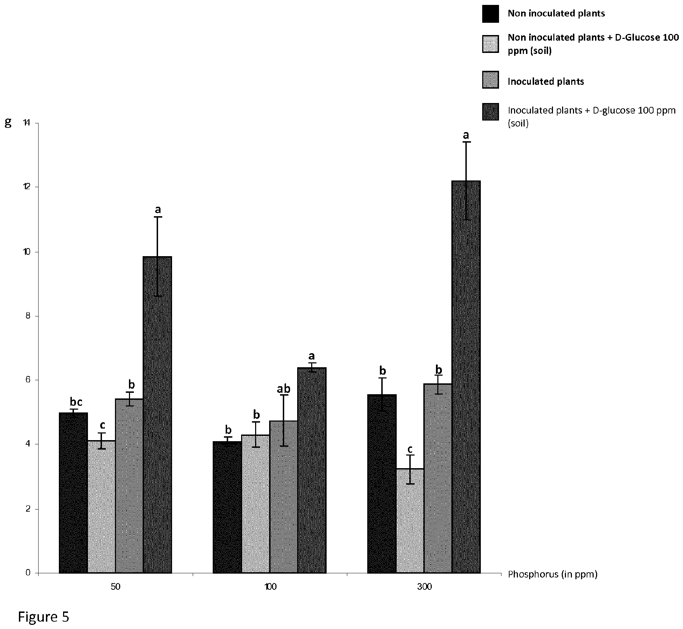

Data on tuber yield obtained after soil application of D-glucose on potato plants in a soil containing 50, 100 or 300 ppm phosphorus is shown in Figure 5. Presence of mycorrhizal fungi tends to increase tuber yield by 1.09; 1.16 and 1.06 fold respectively in presence of 50, 100 or 300 ppm phosphorus compared to non-inoculated plants (not significant). Presence of mycorrhizal fungi with a D-glucose soil application allows to increase significantly the tuber yield by 1.97; 1.56 and 2.20 fold respectively in presence of 50, 100 or 300 ppm phosphorus compared to non-inoculated plants, and by 1.81; 1.35 (non-significant) and 2.08 fold respectively in presence of 50, 100 or 300 ppm phosphorus compared to inoculated plants without D-glucose application. D-glucose soil application in non-inoculated plants decreases potato yield compared to control plant group under 50 ppm phosphorus (1.21 fold, non-significant) and under 300 ppm phosphorus (1.72 fold, significant), while the yield remain stable under 100 ppm phosphorus (increased by 1.05 fold, not significant).

### Conclusion

Application of monosaccharide enhances the mycorrhizal development and potato yield when applied in soil or on leaves, whatever the phosphorus concentration tested. It is advised however to check the pH for a liquid monosaccharide solution and adjust it at 7, especially in the case of foliar application.

## Claims

1. A use of monosaccharides and/or disaccharides for stimulation of mycorrhization in field crop and hydroponic plants whereby the monosaccharides and/or disaccharides are used in a concentration of 100 ppm or less, like between 0.1 to 100 ppm.

2. A use of monosaccharides and/or disaccharides in the presence of mycorrhizal inoculum for improving yield of field crop and hydroponic plants whereby the monosaccharides and/or disaccharides are used in a concentration of 100 ppm or less, preferentially between 0.1 to 100 ppm.

3. The use according to any one of claims 1 to 2, wherein the monosaccharides and/or disaccharides are selected from glucose or fructose.

4. The use according to any one of the preceding claims wherein the monosaccharides and/or disaccharides are applied foliarly.

5. The use according to any one of the preceding claims wherein the monosaccharides and/or disaccharides are applied on or at the vicinity of the roots of the plants.

6. The use according to any one of the preceding claims wherein the monosaccharides and/or disaccharides are applied simultaneously, seperately or sequentially together with mycorrhizal inoculum.

7. The use according to any one of the preceding claims, wherein the monosaccharides and/or disaccharides are applied to mycorrhizal field crop and hydroponic plants on the roots, in the vicinity to the roots or foliarly.

8. The use according to any one of claims 2, 4 to 8, wherein the mycorrhizal inoculum contains spores and/or mycorrhizal roots and/or mycelium.

9. The use according to any one of the preceding claims wherein a fertiliser is applied together with the monosaccharides and/or disaccharides.

10. The use according to any one of the preceding claims for increasing stress tolerance of plants, in particular, when applying monosaccharides and/or disaccharides in combination with mycorrhizal inoculum.

11. A method for the production of mycorrhiza inoculum in plants wherein monosaccharides and/or disaccharides are present in a concentration of 100 ppm or less, like between 0.1 to 100 ppm when producing the mycorrhizal inoculum.

12. A method for treating field crop and hydroponic plants, comprising the step of applying monosaccharides and/or disaccharides and mycorrhizal inoculum on said plants, whereby application of monosaccharides and/or disaccharides and mycorrhiza inoculum may be simultaneously, separately or sequentially and the monosaccharides and/or disaccharides are applied in a concentration of 100 ppm or less, like in between 0.1 to 100 ppm.

13. The use of a composition for stimulating mycorrhization of crop plants, for the production of mycorrhizal inoculum in plants, for increasing the yield of the field crop and hydroponic plants wherein said composition contains monosaccharides and/or disaccharides for application on plant components and mycorrhizal inoculum for mycorrhization of plants according to any one of claims 1 to 12.

## Patentansprüche

1. Verwendung von Monosacchariden und/oder Disacchariden zur Stimulation der Mykorrizierung in Feldfrüchten und hydroponischen Pflanzen, wobei die Monosaccharide und/oder Disaccharide in einer Konzentration von 100 ppm oder weniger, wie zwischen 0,1 und 100 ppm, verwendet werden.

2. Verwendung von Monosacchariden und/oder Disacchariden in Gegenwart von Mykorrhiza-Inokulum zur Verbesserung des Ertrages von Feldfrüchten und hydroponischen Pflanzen, wobei die Monosaccharide und/oder Disaccharide in einer Konzentration von 100 ppm oder weniger, vorzugsweise zwischen 0,1 und 100 ppm, verwendet werden.

3. Die Verwendung nach einem der Ansprüche 1 bis 2, worin die Monosaccharide und/oder Disaccharide ausgewählt sind aus Glukose oder Fruktose.

4. Die Verwendung nach einem der vorherigen Ansprüche, worin die Monosaccharide und/ oder Disaccharide foliar angewendet werden.

5. Die Verwendung nach einem der vorherigen Ansprüche, wobei die Monosaccharide und/ oder Disaccharide auf oder in der Nähe der Wurzeln der Pflanzen aufgebracht oder eingebracht werden.

6. Die Verwendung nach einem der vorherigen Ansprüche, wobei die Monosaccharide und/oder Disaccharide gleichzeitig, getrennt oder aufeinanderfolgend zusammen mit dem Mykorrhiza-Inokulum aufgebracht oder eingebracht werden.

7. Die Verwendung nach einem der vorherigen Ansprüche, wobei die Monosaccharide und/ oder Disaccharide auf Mykorrhiza-Feldfrüchten und hydroponischen Pflanzen an den Wurzeln, in der Nähe der Wurzeln oder foliar aufgebracht werden.

8. Die Verwendung nach einem der Ansprüche 2, 4 bis 8, wobei das Mykorrhiza-Inokulum Sporen und/oder Mykorrhiza-Wurzeln und/oder Myzel enthält.

9. Die Verwendung nach einem der vorherigen Ansprüche, wobei ein Düngemittel zusammen mit den Monosacchariden und/oder Disacchariden aufgebracht wird.

10. Die Verwendung nach einem der vorherigen Ansprüche zur Erhöhung der Stresstoleranz von Pflanzen, insbesondere, wenn die Monosaccharide und/oder Disaccharide in Kombination mit Mykorrhiza-Inokulum aufgebracht wird.

11. Verfahren zur Herstellung von Mykorrhiza-Inokulum in Pflanzen, wobei Monosaccharide und/oder Disaccharide in einer Konzentration von 100 ppm oder weniger, wie zwischen 0,1 bis 100 ppm bei der Herstellung des Mykorrhiza.-Inokulum vorhanden sind.

12. Verfahren zur Behandlung von Feldfrüchten und hydroponischen Pflanzen, umfassend den Schritt des Auftragens von Monosacchariden und/oder Disacchariden und Mykorrhiza-Inokulum auf die Pflanzen, wobei die Anwendung von Monosacchariden und/oder Disacchariden und Mykorrhiza-Inokulum gleichzeitig, separat oder sequentiell erfolgen kann und die Monosaccharide und/oder Disaccharide in einer Konzentration von 100 ppm oder weniger, wie zwischen 0,1 und 100 ppm aufgebracht werden.

13. Die Verwendung einer Zusammensetzung zur Stimulierung der Mykorrhizierung von Kulturpflanzen, zur Herstellung von Mykorrhiza-Inokulum in Pflanzen, zur Erhöhung der Ausbeute bei Feldpflanzen und hydroponischen Pflanzen, wobei die Zusammensetzung Monosaccharide oder Disaccharide enthält zur Anwendung auf Pflanzenkomponenten und Mykorrhiza-Inokulum zur Mykorrhizierung der Pflanzen nach einem der Ansprüche 1 bis 12.

## Revendications

1. Utilisation de monosaccharides et/ou de disaccharides pour la stimulation de la mycorhization chez des plantes hydroponiques et de culture en plein champ, dans laquelle les monosaccharides et/ou les disaccharides sont utilisés selon une concentration de 100 ppm ou moins, telle qu'allant de 0,1 à 100 ppm.

2. Utilisation de monosaccharides et/ou de disaccharides en présence d'inoculum mycorhizien, afin d'augmenter le rendement de plantes hydroponiques et de culture en plein champ, dans laquelle les monosaccharides et/ou les disaccharides sont utilisés selon une concentration de 100 ppm ou moins, allant de préférence de 0,1 à 100 ppm.

3. Utilisation selon l'une quelconque des revendications 1 et 2, dans laquelle les monosaccharides et/ou les disaccharides sont choisis parmi le glucose ou le fructose.

4. Utilisation selon l'une quelconque des revendications précédentes, dans laquelle les monosaccharides et/ou les disaccharides sont appliqués par voie foliaire.

5. Utilisation selon l'une quelconque des revendications précédentes, dans laquelle les monosaccharides et/ou les disaccharides sont appliqués au niveau ou à proximité des racines des plantes.

6. Utilisation selon l'une quelconque des revendications précédentes, dans laquelle les monosaccharides et/ou les disaccharides sont appliqués de manière simultanée, séparée ou séquentielle, conjointement avec un inoculum mycorhizien.

7. Utilisation selon l'une quelconque des revendications précédentes, dans laquelle les monosaccharides et/ou les disaccharides sont appliqués à des plantes mycorhiziennes hydroponiques et de culture en plein champ, aux racines, à proximité des racines ou par voie foliaire.

8. Utilisation selon l'une quelconque des revendications 2, 4 à 8, dans laquelle l'inoculum mycorhizien contient des spores et/ou des racines mycorhiziennes et/ou du mycélium.

9. Utilisation selon l'une quelconque des revendications précédentes, dans laquelle un fertilisant est appliqué conjointement avec les monosaccharides et/ou les disaccharides.

10. Utilisation selon l'une quelconque des revendications précédentes, pour l'augmentation de la tolérance de plantes vis-à-vis d'un stress, en particulier lors de l'application de monosaccharides et/ou de disaccharides en combinaison avec un inoculum mycorhizien.

11. Méthode de production d'un inoculum mycorhizien chez des plantes, dans laquelle des monosaccharides et/ou des disaccharides sont présents selon une concentration de 100 ppm ou moins, telle qu'allant de 0,1 et 100 ppm, lors de la production de l'inoculum mycorhizien.

12. Méthode de traitement de plantes hydroponiques et de culture en plein champ, comprenant l'étape consistant à appliquer des monosaccharides et/ou des disaccharides et un inoculum mycorhizien auxdites plantes, dans laquelle l'application des monosaccharides et/ou des disaccharides et de l'inoculum mycorhizien peut être faite de manière simultanée, séparée ou séquentielle, et les monosaccharides et/ou les disaccharides sont appliqués selon une concentration de 100 ppm ou moins, telle qu'allant de 0,1 à 100 ppm.

13. Utilisation d'une composition pour la stimulation de la mycorhization chez des plantes de culture, pour la production d'un inoculum mycorhizien chez des plantes, pour l'augmentation du rendement des plantes hydroponiques et de culture en plein champ, dans laquelle ladite composition contient des monosaccharides et/ou des disaccharides pour une application à des composants de plantes et un inoculum mycorhizien pour la mycorhization de plantes selon l'une quelconque des revendications 1 à 12.
